# FASCICULE DE BREVET EUROPEEN

(11) **EP 2 453 934 B1**
(45) Date de publication et mention de la délivrance du brevet: **12.06.2013**
(21) Numéro de dépôt: 10737354.0
(22) Date de dépôt: 16.06.2010
(51) Int. Cl.: A61L 27/32, A61L 27/50, A61L 27/56, A61L 27/12

(54) **PROCEDE D'ACTIVATION DE SURFACE DE BIOMATERIAUX POREUX**
VERFAHREN ZUR OBERFLÄCHENAKTIVIERUNG BEI PORÖSEN BIOMATERIALIEN
METHOD FOR ACTIVATING THE SURFACE OF POROUS BIOMATERIALS

(30) Priorité: 18.06.2009 FR 0954110; 18.06.2009 US 487101
(43) Date de publication de la demande: 23.05.2012
(73) Titulaire: Teknimed, 65502 Vic-en-Bigorre Cedex (FR); Institut National Polytechnique De Toulouse, 31029 Toulouse Cedex 4 (FR); Université Paul Sabatier, 31062 Toulouse (FR)
(72) Inventeur: AUTEFAGE, Hélène, F-31300 Toulouse (FR); CAZALBOU, Sophie, F-31320 Rebigue (FR); COMBES, Christèle, F-31520 Ramonville Saint Agne (FR); REY, Christian, F-31320 Aureville (FR)
(74) Mandataire: Hartmann, Jean-Luc
(86) Numéro de dépôt international: PCT/FR2010/051205
(87) Numéro de publication internationale: WO 2010/146312

(56) Documents cités:
- EP-A1- 0 437 975
- EP-A2- 1 384 524

## Description

La présente invention se rapporte au domaine des implants et prothèses orthopédiques en céramique. Plus précisément, elle concerne un procédé d'activation de surface de biomatériaux poreux par recouvrement d'une couche de nanocristaux apatitiques afin d'augmenter leur réactivité de surface.

Les céramiques phosphocalciques sont apparues dans le domaine des biomatériaux depuis une vingtaine d'années. Elles permettent de pallier les inconvénients des greffes biologiques (autogreffes, allogreffes et xénogreffes) tout en favorisant la reconstruction osseuse. En effet, les autogreffes nécessitent une deuxième opération chirurgicale sur le site donneur ; elles ne permettent, généralement, le comblement que de petits volumes du fait du peu de tissu disponible, ce tissu est parfois de mauvaise qualité, chez les patients agés notamment, et elles sont associées à une certaine morbidité. L'allogreffe entraîne une recolonisation souvent réduite [Enneking W.F., Journal of bone and joint surgery, 73-A, 8, 1123-1141, 1991] et des risques infectieux qui peuvent être responsables des ostéolyses massives rencontrées chez certains patients.

De ce fait, les céramiques de synthèse sont couramment utilisées par les chirurgiens (orthopédiques, maxillo-faciaux, plasticiens ou dentaires), dès lors qu'une perte de substance osseuse nécessite un comblement.

L'hydroxyapatite (HA) et le phosphate tricalcique (TCP) sont les deux phosphates de calcium les plus utilisés dans le domaine des biomatériaux [Li Shihong, De Groot Klaas, Layrolle Pierre, Van Blitterswijk Clamens, De Wijn Joost ; Porous ceramic body, US 6,479,418 (2002)], bien que ces deux phases possèdent des propriétés physico-chilniques très différentes.

L'hydroxyapatite peut être considérée comme l'un des phosphates de calcium les moins solubles et elle constitue un biomatériau non biorésorbable. De ce fait, l'hydroxyapatite est couramment utilisée pour le recouvrement des prothèses métalliques de manière à accroître la biointégration du matériau. Le TCP, lui, beaucoup plus soluble constituera un matériau résorbable qui sera progressivement remplacé par l'os. Cependant sa vitesse de résorption n'est pas modulable ("Bioceramics and their clinical applications", Ed. T. Kokubo, CRC Press, 2008).

Les céramiques biphasiques constituées d'un mélange variable de HA et TCP permettent de moduler la bio-résorbabilité en fonction du taux de TCP utilisé et elles connaissent pour cette raison un grand succès dans le domaine des biomatériaux ("Bioceramics and their clinical applications", Ed. T. Kokubo, CRC Press, 2008).

Toutes ces céramiques sont obtenues par frittage à haute température et l'activité biologique de ce type de matériau est limitée en raison de la faible surface spécifique des matériaux frittés et de leur faible interaction avec les diverses protéines et facteurs de croissance responsables de l'adhérence, de la prolifération et de l'expression cellulaire. Elles sont par ailleurs assez éloignées du minéral osseux et se comportent différemment tant sur le plan chimique que biologique.

La plupart des procédures de dépôts d'apatites nanocristallines connues font appel à des solutions sursaturées de phosphate de calcium difficiles à utiliser industriellement, avec des durées de traitement de plusieurs jours parfois. La méthode la plus utilisée consiste à utiliser le « Simulated Body Fluid » (SBF) (Kokubo T, Takadama H (2006), How useful is SBF in predicting in vivo bone bioactivity, Biomaterials 27, 2907-2915). D'autres methodes, sur le même principe, ont été développées utilisant des solutions plus concentrées (Layrolle P, Stigter M, De Groot K, Liu Y, Method for applying a bioactive coating on a médical device, US 6,994,883 (2006), et Layrolle P, de Groot K, de Bruijn J, van Blitterswijk C, Huipin Y, Method for coating médical implants, US 6,733,503 (2004) continuation de US 6,207,218 (2001) ; et également Li P, Wen H B, Hippensteel E, Biological agent-containing ceramic coating and method, US, 7,087,086 (2006) et Li P, Bioactive ceramic coating and method, US 6,569,589 (2003)). Ces méthodes ont par ailleurs été assez peu utilisées pour revêtir l'intérieur des pores d'une céramique.

EP 0 437 975 A1 décrit une méthode permettant de recouvrir un substrat avec un film d'hydroxyapatite. La méthode comprend une étape de préparation d'une dispersion colloïdale floculée d'hydroxyapatite, une étape de recouvrement du substrat par application de la dispersion puis une étape de séchage en deux temps : d'abord à une température inférieure à 40°C puis à une température comprise entre 100 et 200°C. Cette méthode rend possible le recouvrement d'un implant à la forme difficile (notamment les implants poreux) et l'obtention d'une excellente adhésion du revêtement sans étape de calcination afin d'éviter une dégradation de l'implant à cause de la température. Cette méthode est possible grâce à la dispersion colloïdale floculée d'hydroxyapatite. L'hydroxyapatite est de préférence obtenue par un procédé en solution afin d'obtenir des particules plus fines et n'est de préférence pas séchée afin d'éviter la formation d'agglomérats.

Il n'existe ainsi pas de procédés simples et rapides permettant une activation biologique de la surface de céramiques poreuses frittées. Les brevets précités n'ont pas reçu d'application industrielle à ce jour. Par ailleurs, les apatites obtenues par ces procédés sont généralement matures et ont une réactivité moindre que celles précipitées et déposées par le présent procédé, qui offre la possibilité de choisir le temps de maturation.

La présente invention vise à résoudre les inconvénients de l'art antérieur en proposant un procédé permettant de déposer, à basse température, sur des biomatériaux présentant une porosité interconnectée une phase minérale bioactive, d'épaisseur variable, résorbable, constituée de nanocristaux de phosphates de calcium analogues au minéral osseux. Le procédé selon l'invention comporte une imprégnation du matériau poreux par une suspension de phosphate de calcium bioactif suivie d'un séchage dans des conditions définies.

Ce procédé permet le dépôt de nanocristaux très réactifs qui augmentent considérablement la réactivité de surface des céramiques et permettent l'adsorption de substances bioactives capables d'orienter l'activité cellulaire. Le principal avantage de ce procédé est d'activer la surface de matériaux frittés de faible surface spécifique et peu réactifs.

Le procédé selon l'invention s'appuie sur les propriétés de surface des nanocristaux et leur capacité à se fixer sur certaines surfaces, notamment celles de céramiques phosphocalciques poreuses. Ce procédé est simple et efficace et il n'implique aucune manipulation délicate ou coûteuse. Comme explicité ci-après, les gels peuvent être obtenus par la méthode de double décomposition couramment utilisée dans l'industrie. Leur composition et leur viscosité peuvent être parfaitement connues et maîtrisées. Ainsi, les caractéristiques du gel (et par conséquent du dépôt) sont parfaitement adaptées aux caractéristiques du inatériau à traiter. Le procédé fournit un dépôt d'apatites nanocristallines permettant une activation biologique de manière intrinsèque ou par l'utilisation d'ions minéraux bioactifs, de molécules actives soit des deux.

L'invention propose donc un procédé permettant une activation de la surface de ces céramiques par un dépôt de phosphate de calcium apatitique analogue au minéral osseux. Ce dépôt de très grande surface spécifique (Jusqu'à 300 m²/g) permet d'accroître la réactivité superficielle du matériau et facilite l'adsorption de substances bioactives capables d'orienter l'activité cellulaire. Ce procédé de traitement de surface permet de recouvrir la totalité de la surface de la céramique, y compris notamment les surfaces internes des pores interconnectés du matériau.

La phase minérale déposée est constituée de nanocristaux analogues à ceux qui constituent le minéral osseux, Ceux-ci sont caractérisés par la présence, en surface, d'une couche hydratée, labile, très réactive, constituée d'ions facilement et rapidement mobilisables [Cazalbou S., « Echanges cationiques impliquant des apatites nanocristallines analogues au minéral osseux », Thèse INPT, Toulouse 2000]. Ces ions sont capables de participer à de nombreuses réactions d'échanges ioniques et certains peuvent éventuellement avoir une activité biologique. Cette couche hydratée facilite en outre une meilleure interaction avec les molécules organiques du vivant (protéines, facteurs de croissance, ...) [Midy V, Rey C, Bres E, Dard M, Basic fibroblast growth factor adsorption and release of calcium phosphate, Journal of Biomed. and Mat. Res., 405-411, 1998]. Ces environnements labiles semblent conférer aux cristaux des os toute leur réactivité superficielle. On les retrouve en grande quantité dans le minéral osseux, en plus grande quantité dans les os jeunes que dans les os plus matures [Boivin G, Deloffre P, Perrat B, Panczer G, Boudeulle M, Mauras Y, Allain P, Tsouderos Y Meunier P J, Strontium distribution and interactions with bone mineral in monkey iliac bone after strontium salt (S 12911) administration, J. Bone and Min. Res. 11, 9, 1302-1311, 1996]. De plus, la similitude entre la phase déposée et le minéral osseux tant en ce qui concerne la composition chimique que la morphologie cristalline, est susceptible de favoriser une parfaite biointégration.

Un autre avantage de la méthode réside dans la possibilité d'incorporer à l'apatite soit au moment de sa formation, soit après sa formation des ions minéraux bioactifs tels les ions strontium, magnésium, manganèse, vanadate... qui peuvent rester mobilisables et actifs lorsqu'ils sont incorporés à la couche hydratée.

De même l'association de protéines ou de facteurs de croissance favorisant l'ostéogénèse ou la vascularisation peut être réalisée au cours du procédé de revêtement ou bien après dépôt de la couche apatitique nanocristalline. L'association de céramiques phosphocalciques avec des facteurs de croissance tels que les Bone Morphogenetic Proteins (BMP), par exemple, conférère au matériau des propriétés ostéoinductrices [Yuan H, Zou P, Yang Z, Ahang X, De Bruijin J D, De Groot K, Bone morphogenetic protein and ceramic-induced osteogenesis, J. Mater. Sci. Mater. Med.; 9, 12, 717-21;1998*]* et facilite ainsi la cicatrisation osseuse. Le revêtement d'apatite nanocristalline permet d'améliorer les propriétés d'adsorption du matériau vis-à-vis de telles protéines.

L'association de substances bioactives aux céramiques devient possible après dépôt de la couche apatitique nanocristalline (ions, protéines, facteurs de croissance, hormones de croissance...). Il est alors possible d'agir sur le comportement biologique du matériau. C'est dans ce sens que le procédé peut être décrit comme une activation de la surface des céramiques.

Il est, par ailleurs, envisagé que les propriétés accrues du revêtement d'apatite nanocristalline au regard de l'adsorption de protéines et des échanges ioniques puissent faciliter des associations, directement au niveau du site chirurgical, avec les facteurs circulants biologiquement actifs.

Plus précisément, la présente invention concerne un procédé d'activation de surface de biomatériaux poreux caractérisé en ce qu'il comprend les étapes suivantes :
a) préparer un phosphate de calcium apatitique nanocristallin analogue au minéral osseux par mélange d'une solution de sel de calcium avec une solution de sel de phosphate dans un rapport Ca/P compris entre 1,3 et 2, à une température comprise entre 0 et 60°C,
b) mettre en suspension le mélange obtenu à l'étape a) dans une solution aqueuse de manière à obtenir une pâte homogène, fluide contenant 80 à 98 % d'eau,
c) mettre en contact un biomatériau poreux avec la suspension obtenue à l'étape b),
d) sécher le biomatériau poreux à une température inférieure à 100 °C.

La phase nanocristalline de phosphate de calcium analogue au minéral osseux est obtenue par double décomposition entre une solution d'un sel de calcium (pouvant éventuellement contenir d'autres ions, notamment des ions biologiquement actifs) et une solution d'un sel de phosphate et d'un sel de carbonate (pouvant éventuellement contenir d'autres ions, notamment des ions biologiquement actifs). Le principal avantage de cette méthode de synthèse réside dans le fait que le pH reste constant, la solution de synthèse étant tamponnée par un excès de sel de phosphate. De plus, cette méthode évite l'apparition de phase étrangère au cours de la précipitation et limite les phénomènes de dissolution-précipitation. Elle a une excellente reproductibilité. Le précipité obtenu est lavé et récupéré par filtration.

Le phosphate de calcium apatitique nanocristallin ainsi obtenu possède une réactivité de surface contrôlée. Il présente en effet une couche hydratée superficielle comportant des ions mobiles dont l'étendue et la composition sont contrôlés par la maturation des nanocristaux dans la solution de précipitation et/ou la présence d'additifs (Mg²⁺ , CO₃²⁻, P₂O₇⁴⁻) dans cette solution de précipitation.

Dans un mode de réalisation particulier du procédé objet de l'invention, le mélange obtenu à l'issue de l'étape a) est soumis à une étape de traitement de modification de la surface des nanocristaux au cours de laquelle il est mis en contact avec au moins un composé présentant une activité biologique et/ou modifiant les propriétés de surface des nanocristaux, ledit composé étant choisi parmi des ions minéraux ou des molécules organiques ou un mélange des deux. Le traitement de surface peut également être réalisé à l'issue de l'étape d), cumulativement à l'étape a) ou de manière exclusive.

Au cours de cette étape de traitement de surface, le précipité de nanocristaux pourra donc présenter des propriétés de surface définies par l'ajout soit d'ions minéraux soit de molécules organiques, soit d'une combinaison des deux.

Le précipité humide obtenu peut ainsi être soumis à un échange ionique par contact durant quelques minutes avec une solution aqueuse contenant des ions présentant une activité biologique et/ou modifiant les propriétés de surface des nanocristaux -comme par exemple les ions Mg²⁺, Sr²⁺, Mn²⁺, SiO₄⁴⁻ , VO₄³⁻ ou un mélange de ceux-ci. Le précipité modifié obtenu est lavé et récupéré par filtration.

Il est également possible à l'issue de l'étape a), d'associer le précipité avec des molécules biologiquement actives et/ou modifiant les propriétés de surface des nanocristaux, par adsorption de ces dernières sur la surface minérale très réactive, comme par exemple des facteurs de croissance, antibiotiques, ou un mélange de ceux-ci.

Au cours de l'étape b), le précipité est mis en suspension dans une solution aqueuse de manière à obtenir une pâte fluide contenant entre 80 % et 98 % d'eau. La densité de la suspension détermine directement l'épaisseur du dépôt.

De préférence, le mélange obtenu à l'issue de l'étape a) présente un rapport Ca/P comprise entre 1,33 et 1,67 ce qui améliore les propriétés de biointégration ainsi que la réactivité du matériau poreux.

Dans un mode particulier de réalisation de l'invention, avant sa mise en contact avec le gel d'apatite nanocristalline, le biomatériau poreux peut être débarrassé de toute impureté ou polluants organiques éventuels pouvant gêner la mouillabilité de la surface poreuse par la suspension aqueuse par exemple par calcination/chauffage préalable quelques minutes (de 3 à 5 minutes) à l'air, à une température voisine de 900°C, ou encore par un traitement ozonisant sous UV, ou tout autre traitement permettant l'élimination de polluants organiques de surface.

Le matériau poreux à traiter est alors mis en contact avec la suspension. Cette étape peut être réalisée par immersion du biomatériau dans la solution obtenue à l'étape b) ou encore pulvérisation ou application de la solution obtenue à l'étape b) sur le biomatériau. La pénétration du dépôt peut être facilitée par la mise sous vide partiel de l'ensemble, typiquement entre 10 à 30 mm de Hg ; l'air occlus dans les pores est éliminé et remplacé par la suspension. Cette procédure peut être répétée.

Le matériau est ensuite séché à une température n'excédant pas 100°C et préférablement à basse température dans une étuve ventilée ou sous vide partiel.

Lors de cette phase de séchage, les nanocristaux se déposent sur les parois des pores et forment un revêtement. Des craquelures peuvent toutefois apparaître au sein de ce revêtement. Afin d'obtenir un dépôt plus régulier et plus adhérent, il est possible d'inclure au procédé une étape permettant l'épaississement du gel par déshydratation. Cette déshydratation partielle et progressive peut être effectuée de diverses manières par exemple en contrôlant la pression partielle de vapeur d'eau dans la phase gazeuse au contact du matériau, ou en utilisant un solvant par exemple de l'éthanol. Le matériau, après imprégnation, est placé par exemple dans une enceinte fermée en présence de vapeur d'éthanol à une température voisine de 60°C pendant au moins 24 heures puis récupéré et séché comme précédemment. Cette étape influe sur la maturation des nanocristaux ainsi que sur la morphologie du revêtement, qui apparaît plus homogène avec moins de craquelures.

Les surfaces ainsi préparées peuvent adsorber des principes actifs, notamment des facteurs de croissance, des antibiotiques, des médicaments permettant de lutter contre les maladies osseuses (ostéoporose par exemple). L'adsorption est réalisée par contact entre la céramique « activée » et la solution contenant le principe actif. Cette méthode d'association peut se superposer ou se substituer au traitement de surface des nanocristaux réalisé à l'issue de l'étape a).

Les étapes b), c), et d) peuvent être répétées si nécessaire. Les caractéristiques du gel et/ou le nombre de traitements permettent d'obtenir des épaisseurs de dépôts variables typiquement de l'ordre de 1 à 10 µm.

Une répétition modulée, c'est-à-dire effectuée en modifiant le traitement de surface, permet également d'obtenir des dépôts successifs présentant des propriétés biologiques complémentaires, par exemple un dépôt facilitant le recrutement et la croissance des cellules ostéoblastiques qui construisent l'os et contenant des ions strontium, suivi d'un dépôt favorisant la vascularisation et contenant le facteur de croissance VEGF. Ces dépôts successifs en "pelure d'oignon" permettent de contrôler et de favoriser les processus de réparation tissulaire (vascularisation de l'implant, puis sa recolonisation par le tissu osseux). Les épaisseurs sont néanmoins limitées par la nécessité de ne pas altérer significativement ou colmater les pores du matériau.

Un procédé selon l'invention comporte donc la formation d'une couche d'apatite nanocristalline par imprégnation d'un gel. Le principal avantage de ce procédé est d'apporter une activation biologique à des matériaux frittés à base de phosphate de calcium de manière simple et à faible coût.

Avantageusement et selon l'invention, il est possible de faire varier l'épaisseur du dépôt par un contrôle de la viscosité du gel utilisé lors du traitement.

Avantageusement et selon l'invention, la surface spécifique du matériau traité, et sa réactivité superficielle peuvent être considérablement accrue par un contrôle des propriétés physico-chimiques des cristaux constituants le gel (notamment la maturation, ou les modifications de surface).

Avantageusement et selon l'invention, la morphologie du dépôt ainsi que son état de surface peuvent être modifiés.

Avantageusement et selon l'invention, le mode de séchage permet d'améliorer l'adhérence du revêtement au substrat.

Le procédé ainsi décrit selon la présente invention se distingue de celui décrit dans le brevet français FR 2 842 750, "Procédé permettant de recouvrir à basse température des surfaces par des phosphates apatitiques nanocristallins, à partir d'une suspension aqueuse de phosphate amorphe", sur plusieurs points :
(i) Le procédé selon l'invention fait l'économie du passage par un intermédiaire, le phosphate amorphe, pour aboutir à un revêtement nanocristallin. Cette simplicité permet d'éviter les problèmes de contrôle de la germination et de la croissance cristalline de la phase amorphe évoqués dans le premier brevet.
(ii) Le procédé selon l'invention offre en outre la possibilité de faire varier dans un large domaine le rapport Ca/P de la phase nanocristalline et en conséquence, ses propriétés biologiques, notamment sa capacité à se résorber plus ou moins vite en milieu biologique. Cette variation peut être directement obtenue en jouant sur les conditions de synthèse de l'apatite notamment, le pH, l'ordre d'addition des réactifs, la température, la présence d'ions étrangers dans la solution de synthèse (par exemple carbonate). Les apatites directement obtenues peuvent ainsi présenter une large gamme de rapports Ca/P (1,30 à 2) liée à la proportion d'ions HPO₄²⁻ et/ou carbonate incorporés dans la phase nanocristalline au moment de sa synthèse.
(iii) Un autre avantage du procédé de recouvrement selon l'invention tient à la possibilité de contrôler directement la couche hydratée de l'apatite synthétisée de façon à favoriser l'adhérence des cristaux sur le substrat, et à contrôler leur caractéristiques de surface, et la réponse cellulaire. Ces contrôles s'effectuent en jouant sur le temps de maturation (temps de vieillissement des cristaux en solution) et la présence de stabilisants de cette couche hydratée notamment les ions minéraux tels que les ions carbonate, pyrophosphate, Mg²⁺ ou encore des molécules organiques. Ces adjuvants peuvent être associés aux solutions de synthèse ou incorporés à la couche hydratée après la formation de l'apatite nanocristalline.
(iv) Un autre avantage du procédé selon l'invention est de disposer d'une population homogène de nanocristaux, qu'il est impossible d'obtenir par le procédé d'hydrolyse selon FR 284 2750, relativement lent, de la phase amorphe qui conduit, nécessairement, à un mélange de cristaux de maturité différente.

Les propriétés et avantages du procédé objet de la présente invention apparaîtront plus clairement à la lueur des exemples qui suivent. Ceux-ci sont donnés à titre purement illustratif et ne doivent pas être interprétés comme une quelconque limitation à la portée du procédé qui s'étend notamment aux moyens équivalents à ceux qui sont décrits dans la présente demande.

Les figures suivantes servent également à illustrer la présente invention :
- Figure 1 : Micrographie d'une couche d'apatite nanocristalline déposée à l'intérieur des pores d'une céramique biphasique HA-TCP selon l'invention.
- Figure 2 : Spectre infrarouge des nanocristaux déposés au cours du procédé de l'exemple 1 selon l'invention.
- Figure 3 : Diagramme des diffractions des rayons X des nanocristaux déposés au cours du procédé de l'exemple 1 selon l'invention (Radiation CoK_{α}).

### EXEMPLES

### Exemple 1 : dépôt d'apatite carbonatée nanocristalline proche des apatites biologiques

Etape 1 : Synthèse du gel d'apatite carbonatée
   Solution A : 48,8 g Na₂HPO₄. 12H₂O + 18 g NaHCO₃ dans 400 ml d'eau désionisée
   Solution B : 6,5 g CaCl₂.2H₂O dans 150 ml d'eau désionisée
      Après dissolution complète des sels dans les solutions A et B, verser la solution B dans la solution A. Puis filtrer et rincer soigneusement à l'eau désionisée.
Etape 2 : Remettre 50 g de gel dans 200 ml d'eau de manière à obtenir une suspension homogène. Cette suspension constituera la solution C.
Etape 3 : Une céramique poreuse de phosphate de calcium (cube de 30 mm³, 70 % porosité interconnectée) est plongée dans la solution C placée dans une fiole à vide.
   On établit le vide durant une dizaine de minutes tout en remuant la solution de manière à éliminer les bulles qui se forment. Puis, on rétablit rapidement la pression atmosphérique ambiante de manière à faire pénétrer le gel à l'intérieur des pores de la céramique. Cette étape est répétée à plusieurs reprises si nécessaire.
Etape 4 : Séchage de la céramique
   La céramique est placée dans une enceinte fermée en présence de vapeur d'éthanol à 60°C pendant 24 heures afin d'éliminer une partie de l'eau contenue dans le gel et ainsi limiter l'apparition de craquelures lors du séchage.
   La céramique ainsi traitée est séchée dans une enceinte appropriée à 4°C durant 48 heures.

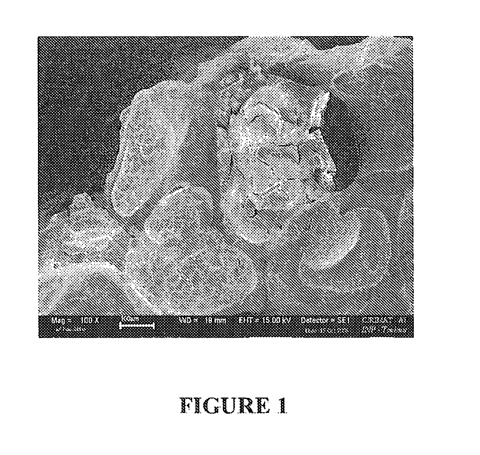

   La figure 1 représente le dépôt obtenu et les figures suivantes, ses principales caractéristiques physico-chimiques. L'analyse chimique donne un rapport atomique Ca/P de 1,50 et une teneur en carbonate de 1,50 % en masse. Ces données caractérisent une apatite non-stoechiométrique fortement lacunaire.

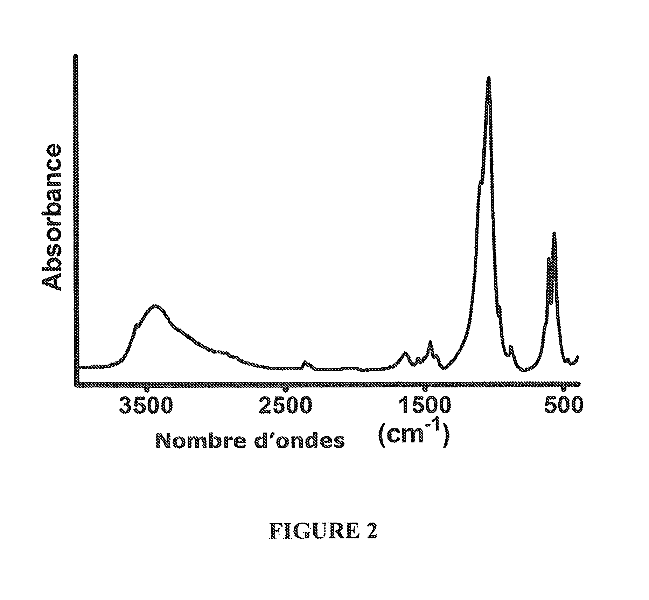

On remarque sur la figure 2 les bandes phosphate caractéristiques d'une apatite phosphocalcique (v₁ PO₄ : 961 cm⁻¹, v₂ PO₄ : 460 cm⁻¹, v₃ PO₄ : 1030 et 1095 cm⁻¹, v₄ PO₄ : 560 et 600 cm⁻¹). Les bandes carbonates (v₂ CO₃ : 860-890 cm⁻¹ et v₃ CO₃ : 1400-1550 cm⁻¹) montrent une substitution à la fois des ions phosphate (carbonates de type B) et des ions OH⁻(carbonates de type A). On notera aussi la présence d'ions OH en faible proportion (épaulements vers 3570 et 630 cm⁻¹).

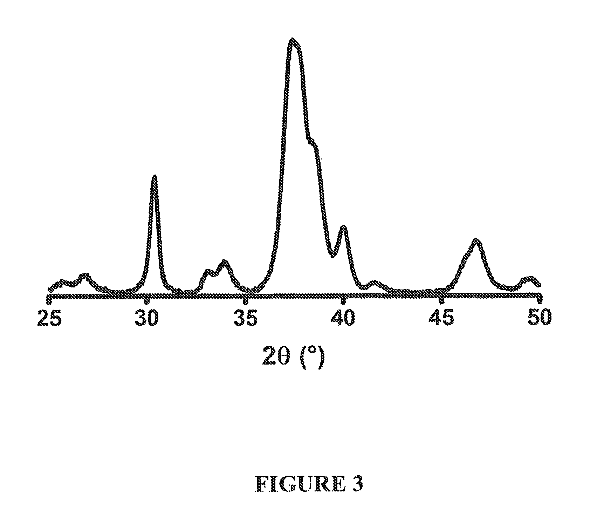

La figure 3 permet d'observer un diagramme caractéristique d'une apatite. Aucune phase étrangère cristallisée n'est détectée. Les dimensions cristallines déduites de ces diagrammes par la formule de Scherrer (longueur : 24,5 ± 0,5 nm et largeur-épaisseur : 10,2 ± 0,3 nm) confirment la nature nanocristalline des cristaux.

### Exemple 2 : dépôt d'apatite non-carbonatée nanocristalline très riche en ions HPO₄²⁻, de rapport Ca/P proche de 1,35 et présentant une partie importante des ions minéraux dans la couche hydratée

Etape 1 : Synthèse du gel d'apatite
   Solution A : 40 g (NH₄)₂HPO₄ dans 500 ml d'eau désionisée
   Solution B : 17,4 g Ca(NO₃)₂,4H₂O dans 250 ml d'eau désionisée
   Après dissolution complète des sels dans les solutions A et B, verser la solution B dans la solution A. Puis filtrer et rincer soigneusement à l'eau désionisée.
Etape 2 : Remettre 50 g de gel dans 200 ml d'eau de manière à obtenir une suspension homogène. Cette suspension constituera la solution C.
Etape 3 : Une céramique poreuse de phosphate de calcium (cube de 30 mm³, 70 % porosité interconnectée) est plongée dans la solution C placée dans une fiole à vide.
   On établit le vide durant une dizaine de minutes tout en remuant la solution de manière à éliminer les bulles qui se forment. Puis, on rétablit rapidement la pression atmosphérique ambiante de manière à faire pénétrer le gel à l'intérieur des pores de la céramique. Cette étape est répétée à plusieurs reprises si nécessaire.
Etape 4 : Séchage de la céramique
   La céramique est séchée à l'air puis sous vide à température ambiante.
   Le rapport atomique Ca/P pour ce dépôt est proche de 1,35 et sa teneur en ions HPO₄²⁻ est voisine de 29 %. La taille des cristaux est : longueur : 17 nm, largeur épaisseur : 5 nm.

### Exemple 3 : dépôt d'apatite carbonatée nanocristalline de rapport Ca/P proche de 1,6

Etape 1 : Synthèse du gel d'apatite carbonatée.
   Solution A : 48.8 g Na₂HPO₄.12H₂O + 18 g NaHCO₃ dans 400 ml d'eau désionisée
   Solution B : 6.5 g CaCl₂.2H₂O dans 150 ml d'eau désionisée
   Après dissolution complète des sels dans les solutions A et B, verser la solution B dans la solution A. La suspension est laissée à maturer durant plusieurs mois. Puis filtrer et rincer soigneusement à l'eau désionisée.

Les autres étapes sont identiques à celles de l'exemple 1.

Le rapport Ca/P pour ces dépôts (maturation 2 mois) est 1,58; le rapport C/P: 0,14. Longueur des cristaux : 25 nm.

### Exemple 4 : dépôt d'apatite carbonatée nanocristalline dopée au strontium

Etape 1 : Synthèse du gel d'apatite carbonatée
   Solution A : 40 g (NH₄)₂HPO₄ + 20 g NaHCO₃ dans 500 ml d'eau désionisée
   Solution B : 17.7 g CaNO₃ dans 250 ml d'eau désionisée
   Après dissolution complète des sels dans les solutions A et B, verser la solution B dans la solution A. Puis filtrer et rincer soigneusement à l'eau désionisée.
Etape 4 : Mettre 100 g de gel dans 200 ml de solution de nitrate de strontium (0.5 M) durant 10 minutes sous agitation puis filtrer et rincer soigneusement à l'eau désionisée.
Etape 3 : Remettre 100 g de gel dans 400 ml d'eau de manière à obtenir une suspension homogène. Cette suspension constituera la solution C.
Etape 4 : Une céramique poreuse de phosphate de calcium (cube de 1 cm³, 80 % porosité interconnectée) est plongée dans la solution C placée dans une fiole à vide.
   On établit le vide durant une dizaine de minutes tout en remuant la solution de manière à éliminer les bulles qui se forment. Puis, on rétablit rapidement la pression atmosphérique ambiante de manière à faire pénétrer le gel à l'intérieur des pores de la céramique. Cette étape est répétée à plusieurs reprises si nécessaire.
Etape 5 : La céramique ainsi traitée est séchée à l'étuve à 50°C durant 48 heures.
   Les taux de substitution du calcium par le strontium peuvent varier de 0 à 20 % selon la concentration des solutions d'échange et l'état de maturation de l'apatite.

### Exemple 5 : dépôt d'apatite carbonatée nanocristalline associant un facteur de croissance

Les étapes 1-5 de l'exemple 1 sont inchangées. Ensuite la céramique revêtue est plongée dans une solution de facteur de croissance rhBMP-2. Le facteur se fixe alors sur la céramique activée en proportion notablement plus importante que sur la céramique non-activée (517 µg/g au lieu de 397 µg/g).

## Revendications

1. Procédé d'activation de surface de biomatériaux poreux ***caractérisé en ce qu'***il comprend les étapes suivantes :
a) préparer un phosphate de calcium apatitique nanocristallin analogue au minéral osseux par mélange d'une solution de sel de calcium avec une solution de sel de phosphate dans un rapport Ca/P compris entre 1,3 et 2 à une température comprise entre 0 et 60 °C,
b) mettre en suspension le mélange obtenu à l'étape a) dans une solution aqueuse de manière à obtenir une pâte homogène, fluide contenant 80 à 98 % d'eau,
c) mettre en contact un biomatériau poreux avec la suspension obtenue à l'étape b),
d) sécher le biomatériau poreux à une température inférieure à 100 °C.

2. Procédé selon la revendication 1, ***caractérisé en ce que*** le mélange obtenu à l'issue de l'étape a) est soumis à un traitement de modification de la surface des nanocristaux par mise en contact avec au moins un composé présentant une activité biologique et/ou modifiant les propriétés de surface des nanocristaux, ledit composé étant choisi parmi des ions minéraux ou des molécules organiques ou un mélange des deux.

3. Procédé selon la revendication 1 ou 2, ***caractérisé en ce que*** le biomatériau obtenu à l'issue de l'étape d) est soumis à un traitement de surface par mise en contact avec au moins un composé présentant une activité biologique et/ou modifiant les propriétés de surface des nanocristaux, ledit composé étant choisi parmi des ions minéraux ou des molécules organiques ou un mélange des deux.

4. Procédé selon la revendication 2 ou 3, ***caractérisé en ce que*** le traitement de surface est réalisé par échange ionique avec une solution aqueuse contenant au moins un ion choisi parmi : Mg²⁺, Sr²⁺, Mn²⁺, SiO₄⁴⁻, VO₄³⁻ ou un mélange de ceux-ci.

5. Procédé selon l'une quelconque des revendications 2 à 4, ***caractérisé en ce que*** le traitement de surface est réalisé par adsorption d'au moins une molécule organique choisie parmi : des facteurs de croissance, antibiotiques, ou un mélange de ceux-ci.

6. Procédé selon l'une quelconque des revendications 1 à 5, ***caractérisé en ce que*** le mélange obtenu à l'issue de l'étape a) présente un rapport Ca/P compris entre 1,33 et 1,67.

7. Procédé selon l'une quelconque des revendications 1 à 6, ***caractérisé en ce que*** préalablement à l'étape c), le biomatériau est nettoyé de toute impureté organique pouvant gêner le mouillage par la suspension aqueuse par chauffage préalable à l'air à une température voisine de 900 °C pendant 3 à 5 minutes, ou par traitement d'ozonisation.

8. Procédé selon l'une quelconque des revendications 1 à 7, ***caractérisé en ce que*** l'étape c) est réalisée par immersion du biomatériau dans la solution obtenue à l'étape b) ou encore pulvérisation ou application de la solution obtenue à l'étape b) sur le biomatériau.

9. Procédé selon la revendication 8, ***caractérisé en ce que*** l'étape c) est effectuée sous vide partiel avec une pression comprise entre 10 à 30 mm de Hg.

10. Procédé selon l'une quelconque des revendications 1 à 9, ***caractérisé en ce que**,* préalablement à l'étape d), le biomatériau obtenu à l'issue de l'étape c) est partiellement et progressivement déshydraté.

11. Procédé selon l'une quelconque des revendications 1 à 10, ***caractérisé en ce que*** les étapes b), c) et d) sont répétées.

12. Procédé selon l'une quelconque des revendications 1 à 11, ***caractérisé en ce que*** le biomatériau est une céramique phosphocalcique.

## Patentansprüche

1. Verfahren zur Oberflächenaktivierung bei porösen Biomaterialien, **dadurch gekennzeichnet, dass** es die nachfolgenden Schritte umfasst:
a) Herstellen eines nanokristallinen, apatitischen Calciumphosphats, das analog zum Knochenmineral ist, durch Vermischen einer Calciumsalzlösung mit einer Phosphatsalzlösung in einem Verhältnis Ca/P von zwischen 1,3 und 2 bei einer Temperatur von zwischen 0 und 60 °C,
b) Suspendieren des in Schritt a) erhaltenen Gemisches in einer wässrigen Lösung, so dass eine flüssige, homogene Masse erhalten wird, die 80 bis 98 % Wasser enthält,
c) In-Kontakt-Bringen eines porösen Biomaterials mit der in Schritt b) erhaltenen Suspension,
d) Trocknen des porösen Biomaterials bei einer Temperatur von unter 100 °C.

2. Verfahren nach Anspruch 1, **dadurch gekennzeichnet, dass** das am Ende von Schritt a) erhaltene Gemisch einer Behandlung zum Verändern der Oberfläche der Nanokristalle durch In-Kontakt-Bringen mit zumindest einer Verbindung ausgesetzt wird, die eine biologische Aktivität aufweist und/oder die Oberflächeneigenschaften der Nanokristalle verändert, wobei die Verbindung ausgewählt ist aus Mineral-lonen oder organischen Molekülen oder einem Gemisch von beiden.

3. Verfahren nach Anspruch 1 oder 2, **dadurch gekennzeichnet, dass** das am Ende von Schritt d) erhaltene Biomaterial einer Oberflächenbehandlung durch In-Kontakt-Bringen mit zumindest einer Verbindung ausgesetzt wird, die eine biologische Aktivität aufweist und/oder die Oberflächeneigenschaften der Nanokristalle verändert, wobei die Verbindung ausgewählt ist aus Mineral-lonen oder organischen Molekülen oder einem Gemisch von beiden.

4. Verfahren nach Anspruch 2 oder 3, **dadurch gekennzeichnet, dass** die Oberflächenbehandlung durch lonenaustausch mit einer wässrigen Lösung erfolgt, die zumindest ein Ion, ausgewählt aus Mg²⁺, Sr²⁺, Mn²⁺, SiO₄⁴⁻, VO₄³⁻ oder einem Gemisch derselben, enthält.

5. Verfahren nach einem der Ansprüche 2 bis 4, **dadurch gekennzeichnet, dass** die Oberflächenbehandlung durch Adsorption von zumindest einem organischen Molekül, ausgewählt aus Wachstumsfaktoren, Antibiotika oder einem Gemisch derselben, erfolgt.

6. Verfahren nach einem der Ansprüche 1 bis 5, **dadurch gekennzeichnet, dass** das am Ende von Schritt a) erhaltende Gemisch ein Verhältnis Ca/P von zwischen 1,33 und 1,67 aufweist.

7. Verfahren nach einem der Ansprüche 1 bis 6, **dadurch gekennzeichnet, dass** vor dem Schritt c) das Biomaterial von jeglicher organischer Unreinheit gereinigt wird, die die Benetzung durch die wässrige Lösung beeinträchtigen könnte, und zwar durch vorheriges Erhitzen mit Luft auf eine Temperatur von ca. 900 °C für 3 bis 5 Minuten oder durch eine Behandlung mit Ozonisieren.

8. Verfahren nach einem der Ansprüche 1 bis 7, **dadurch gekennzeichnet, dass** der Schritt c) durch Eintauchen des Biomaterials in die in Schritt b) erhaltene Lösung oder auch durch Aufsprühen oder Auftragen der in Schritt b) erhaltenen Lösung auf das Biomaterial erfolgt.

9. Verfahren nach Anspruch 8, **dadurch gekennzeichnet, dass** der Schritt c) unter einem Teilvakuum mit einem Druck von zwischen 10 bis 30 mm Hg erfolgt.

10. Verfahren nach einem der Ansprüche 1 bis 9, **dadurch gekennzeichnet, dass** vor dem Schritt d) das am Ende von Schritt c) erhaltene Biomaterial teilweise und schrittweise entwässert wird.

11. Verfahren nach einem der Ansprüche 1 bis 10, **dadurch gekennzeichnet, dass** die Schritte b), c) und d) wiederholt werden.

12. Verfahren nach einem der Ansprüche 1 bis 11, **dadurch gekennzeichnet, dass** das Biomaterial Calciumphosphat-Keramik ist.

## Claims

1. Porous biomaterials surface activation method ***characterised in that*** it comprises the following steps:
a) preparing a nanocrystalline apatitic calcium phosphate analogous to bone mineral by mixing a calcium salt solution with a phosphate salt solution in a Ca/P ratio ranging between 1.3 and 2 at a temperature ranging between 0 and 60 °C,
b) slurrying the mixture obtained in step a) in an aqueous solution so as to obtain a fluid, homogeneous paste containing 80 to 98% of water,
c) contacting a porous biomaterial with the suspension obtained in step b),
d) drying the porous biomaterial at a temperature below 100 °C.

2. Method according to claim 1, ***characterised in that*** the mixture obtained once step a) is completed undergoes a treatment to modify the surface of the nanocrystals by bringing it into contact with at least one compound exhibiting a biological activity and/or modifying the surface properties of the nanocrystals, said compound being selected from mineral ions or organic molecules or a mixture thereof.

3. Method according to claim 1 or 2, ***characterised in that*** the biomaterial obtained once step d) is completed undergoes a surface treatment by bringing it into contact with at least one compound exhibiting a biological activity and/or modifying the surface properties of the nanocrystals, said compound being selected from mineral ions or organic molecules or a mixture thereof.

4. Method according to claim 2 or 3, ***characterised in that*** the surface treatment is carried out by ion exchange with an aqueous solution containing at least one ion selected from: Mg²⁺, Sr²⁺, Mn²⁺, SiO₄⁴⁻, VO₄³⁻ or a mixture thereof.

5. Method according to any of claims 2 to 4, ***characterised in that*** the surface treatment is carried out by adsorption of at least one organic molecule selected from: growth factors, antibiotics, or a mixture thereof.

6. Method according to any of claims 1 to 5, ***characterised in that*** the mixture obtained once step a) is completed has a Ca/P ratio ranging between 1.33 and 1.67.

7. Method according to any of claims 1 to 6, ***characterised in that*** prior to step c), the biomaterial is cleared of any organic impurity that could hinder wetting by the aqueous suspension by prior heating in air at a temperature of ca. 900 °C for 3 to 5 minutes, or by ozonization treatment.

8. Method according to any of claims 1 to 7, ***characterised in that*** step c) is carried out by immersion of the biomaterial in the solution obtained in step b) or by spraying or coating the solution obtained in step b) on the biomaterial.

9. Method according to claim 8, ***characterised in that*** step c) is carried out under partial vacuum with a pressure ranging between 10 to 30 mm Hg.

10. Method according to any of claims 1 to 9, ***characterised in that**,* prior to step d), the biomaterial obtained at the end of step c) is partially and progressively dehydrated.

11. Method according to any of claims 1 to 10, ***characterised in that*** steps b), c) and d) are repeated.

12. Method according to any of claims 1 to 11, ***characterised in that*** the biomaterial is a phosphocalcic ceramic.
